(19) Europäisches Patentamt / European Patent Office / Office européen des brevets

(11) **EP 4 293 674 A1**

(12) **EUROPEAN PATENT APPLICATION**

(43) Date of publication:
**20.12.2023 Bulletin 2023/51**

(21) Application number: **23155997.2**

(22) Date of filing: **10.02.2023**

(51) International Patent Classification (IPC):
**G16C 20/30** (2019.01)   **G16B 15/00** (2019.01)
**G16C 20/40** (2019.01)

(52) Cooperative Patent Classification (CPC):
**G16C 20/30; G16B 15/30; G16C 20/40**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.06.2022 JP 2022097375**

(71) Applicant: **FUJITSU LIMITED**
**Kawasaki-shi, Kanagawa 211-8588 (JP)**

(72) Inventor: **Katayama, Kentaro**
**Kawasaki-shi, Kanagawa, 211-8588 (JP)**

(74) Representative: **Hoffmann Eitle**
**Patent- und Rechtsanwälte PartmbB**
**Arabellastraße 30**
**81925 München (DE)**

(54) **STABLE STRUCTURE SEARCH SYSTEM, STABLE STRUCTURE SEARCH METHOD, AND STABLE STRUCTURE SEARCH PROGRAM**

(57)    A stable structure search system including: a calculation unit that, in a model in which N particles (N is an integer equal to or greater than two) arranged in a shape of a row are arranged in a lattice space, calculates coordinates in the lattice space of an i+1-th particle in the row, by using the coordinates in the lattice space of an i-th particle in the row, and state variables represented by relative coordinates in the lattice space of the i-th particle and the i+1-th particle; a computation unit that computes a value of energy of the model, based on the coordinates in the lattice space of each of the N particles, every time the state variables are altered; and a specifying unit that specifies the state variables with which the value of the energy has a local minimum value.

FIG. 1

EP 4 293 674 A1

**Description**

FIELD

The embodiments discussed herein are related to a stable structure search system, a stable structure search method, and a stable structure search program.

BACKGROUND

In recent years, in the field of drug discovery, medium molecules (molecular weight 500 to 3000) with few side effects have attracted attention, and the development of a search method for searching for a stable structure of medium molecules is underway.

As one example, a method of searching for a stable structure in a coarse-grained model using interaction potentials between coarse-grained particles can be mentioned.

International Publication Pamphlet No. WO 2019/230303, Japanese Laid-open Patent Publication No. 2020-194487, Japanese Laid-open Patent Publication No. 2019-179304, and Japanese Laid-open Patent Publication No. 2021-82165 are disclosed as related art.

SUMMARY

TECHNICAL PROBLEM

However, in the case of the above method, when the number of coarse-grained particles becomes greater, the amount of computation increases, making it difficult to find a solution.

An object of one aspect is to reduce the amount of computation when searching for a stable structure of a coarse-grained model.

SOLUTION TO PROBLEM

According to an aspect of the embodiments, there is provided a stable structure search system including: a calculation unit that, in a model in which N particles (N is an integer equal to or greater than two) arranged in a shape of a row are arranged in a lattice space, calculates coordinates in the lattice space of an i+1-th particle in the row, by using the coordinates in the lattice space of an i-th particle in the row, and state variables represented by relative coordinates in the lattice space of the i-th particle and the i+1-th particle; a computation unit that computes a value of energy of the model, based on the coordinates in the lattice space of each of the N particles, every time the state variables are altered; and a specifying unit that specifies the state variables with which the value of the energy has a local minimum value.

ADVANTAGEOUS EFFECTS OF INVENTION

The amount of computation when searching for a stable structure of a coarse-grained model may be reduced.

BRIEF DESCRIPTION OF DRAWINGS

FIG. 1 is a diagram illustrating an application example of a stable structure search system;
FIG. 2 is a diagram illustrating an overview of a stable structure search process for a coarse-grained model;
FIGs. 3A and 3B are diagrams illustrating a representation method with relative coordinates;
FIGs. 4A and 4B are diagrams illustrating a specific example of state variables;
FIG. 5 is a diagram illustrating details of an energy computation process;
FIG. 6 is a first diagram illustrating details of each term of the energy computation process;
FIG. 7 is a second diagram illustrating details of each term of the energy computation process;
FIG. 8 is a diagram illustrating an example of the system configuration of a stable structure search system and the functional configuration of a terminal device;
FIG. 9 is a diagram illustrating an example of the system configuration of the stable structure search system and the functional configuration of an Ising device;
FIG. 10 is a diagram illustrating an example of the hardware configuration of the terminal device;

FIG. 11 is a flowchart illustrating a flow of the stable structure search process;

FIG. 12 is a first flowchart illustrating details of a search process;

FIG. 13 is a second flowchart illustrating details of the search process; and

FIG. 14 is a diagram illustrating an example of the effect of reduction in the amount of computation.

DESCRIPTION OF EMBODIMENTS

Hereinafter, each embodiment will be described with reference to the accompanying drawings. Note that, in the present specification and the drawings, constituent elements having substantially the same functional configuration are given the same reference sign, and redundant description will be omitted.

[First Embodiment]

<Application Example of Stable Structure Search System>

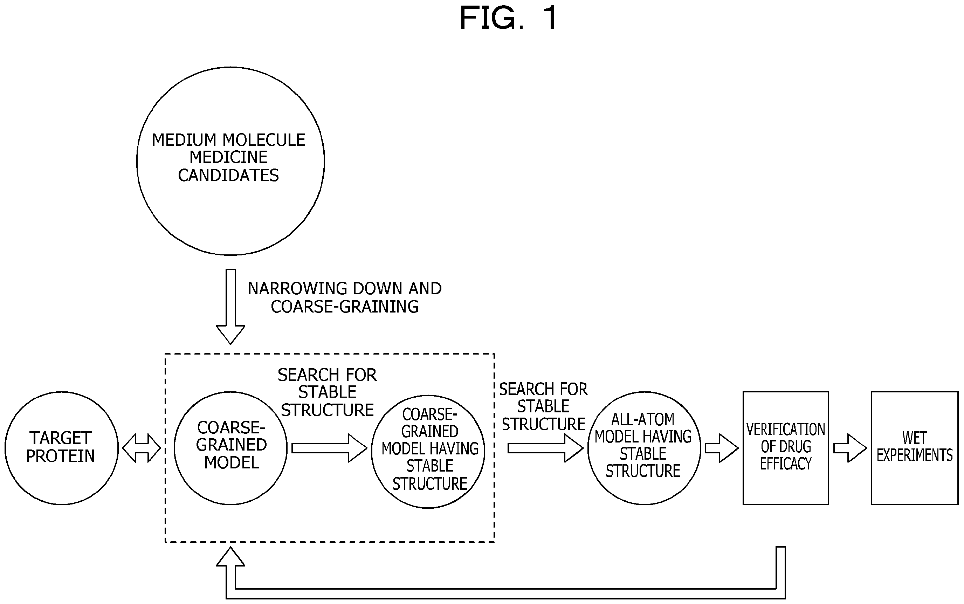

First, an application example of a stable structure search system according to a first embodiment will be described. FIG. 1 is a diagram illustrating an application example of the stable structure search system.

Commonly, in the field of drug discovery, the mainstream so far has been to use low-molecular-weight molecules. However, in the case of the low-molecular-weight molecules, a bind to proteins other than the target protein could easily happen, which has been likely to inhibit functions other than those intended. For this reason, in recent years, medium molecules with less side effects have attracted attention.

Meanwhile, in the case of medicine candidate medium molecules, the amount of computation is enormous when searching for a stable structure. Thus, the applicants of the present application have proceeded with the development of a search method for first narrowing down to specified medium molecules (for example, cyclic peptides) and then searching for a stable structure of the narrowed-down medium molecules in the following distinct two phases.

The first phase is a phase to generate a coarse-grained model (an example of a model) by representing (coarse-graining) an amino acid molecule with one particle and to search for a stable structure of the coarse-grained model. Note that the stable structure search system according to the first embodiment is a system applied to the first phase indicated by the dotted line frame in FIG. 1 and is a system that achieves a reduction in the amount of computation when searching for a stable structure of the coarse-grained model.

The second phase is a phase to search for a stable structure with all atoms based on the coarse-grained model having the stable structure found by the search and to additionally verify the drug efficacy based on the all-atom model having the stable structure found by the search.

Note that, when the preferred drug efficacy is not obtained in the second phase, a search for a further stable structure is made for the coarse-grained model by returning to the first phase. The above cycle is repeatedly performed until the preferred drug efficacy is obtained, and when the preferred drug efficacy is obtained, a medium molecule medicine will be synthesized based on the all-atom model having the stable structure at that time and activity evaluations and the like (so-called wet experiments) will be conducted by, for example, a pharmaceutical manufacturer.

The stable structure search system according to the first embodiment, which will be described in detail below, is a system applied to the first phase of such a comprehensive flow of medium molecule drug discovery.

<Overview of Stable Structure Search Process for Coarse-Grained Model>

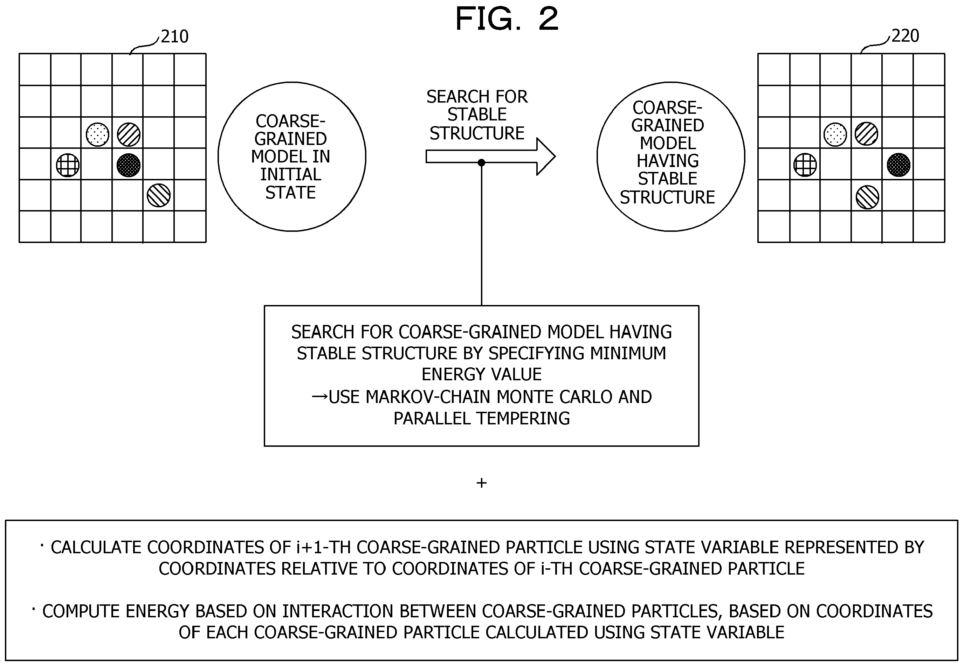

Next, an overview of a search process for a stable structure of a coarse-grained model by the stable structure search system according to the first embodiment will be described. FIG. 2 is a diagram illustrating an overview of the stable structure search process for a coarse-grained model.

In the stable structure search system according to the first embodiment, as an amino acid molecule coupled in a linear chain, · a skeleton portion centered on the $\alpha$-carbon of an amino acid forming a main chain particle of a peptide, and · a side chain particle are each replaced by one particle that is coarse-grained (coarse-grained particle), and · the replaced coarse-grained particles are arranged in a row shape in, for example, a face-centered cubic (FCC) space (lattice space) to thereby generate a coarse-grained model including a plurality of coarse-grained particles, and a search for a stable structure of the generated coarse-grained model is made as a combinatorial optimization problem using an Ising device.

In FIG. 2, the reference sign 210 schematically illustrates a coarse-grained model in an initial state, in which a plurality of coarse-grained particles (five coarse-grained particles in the example in FIG. 2) arranged in a face-centered cubic (FCC) space (lattice space). Note that, in the example in FIG. 2, the space of the face-centered cubic is represented by a two-dimensional plane for simplification of illustration.

As illustrated in FIG. 2, in the stable structure search system according to the first embodiment, when searching for a stable structure of the coarse-grained model (reference sign 210), the state transition of the coarse-grained model is repeated by moving the position of each coarse-grained particle in the space of the face-centered cubic. Then, in the stable structure search system according to the first embodiment, in the course of repeating the state transition, · transition permissibility (also referred to as alterability) is determined every time based on the difference between the energy value before the transition and the energy value after the transition, and · the minimum energy value (more precisely, the energy value having a local minimum value) is specified from among the energy values after transition (after alteration) determined that the transition is permitted (to be alterable). This is how the stable structure search system according to the first embodiment searches for a coarse-grained model (reference sign 220) having a stable structure.

Specifically, the stable structure search system according to the first embodiment uses the Markov-Chain Monte Carlo (MCMC) to · determine that the transition is permitted when the energy value after the transition is smaller than the energy value before the transition, and · determine that the transition is permitted when the difference between the energy value after the transition and the energy value before the transition is smaller than a predetermined thermal noise even when the energy value after the transition is greater than the energy value before the transition (that is, determine that the transition is permitted when a predetermined condition is satisfied), thereby specifying the minimum energy value without falling into a local solution.

In addition, by repeating the state transition of the coarse-grained model using the parallel tempering (PT), the stable structure search system according to the first embodiment efficiently specifies the minimum energy value.

Besides, the stable structure search system according to the first embodiment · calculates the coordinates of the i+1-th coarse-grained particle in a row among a plurality of coarse-grained particles arranged in the row shape, using a state variable represented by coordinates relative to the coordinates of the i-th coarse-grained particle in the row, and · computes the energy value based on the interaction between the coarse-grained particles, based on the coordinates of each coarse-grained particle calculated using the state variable, thereby specifying the minimum energy value with a small amount of computation (details will be described later). The "state variable" mentioned here is a variable for specifying the state of the coarse-grained model (the coordinates of each coarse-grained particle of the coarse-grained model).

Note that, in FIG. 2, the reference sign 220 schematically illustrates a stable structure of the coarse-grained model found by the search in response to the minimum energy value specified by the stable structure search system according to the first embodiment. However, as with the reference sign 210, the reference sign 220 represents the space of the face-centered cubic in a two-dimensional plane for simplification of illustration.

<Advantages of Representing State Variables by Relative Coordinates>

As described above, in the stable structure search system according to the first embodiment, the coordinates of the i+1-th coarse-grained particle are calculated using a state variable represented by coordinates relative to the coordinates of the i-th coarse-grained particle. Thus, in the following, the advantages of representing state variables by relative coordinates will be described.

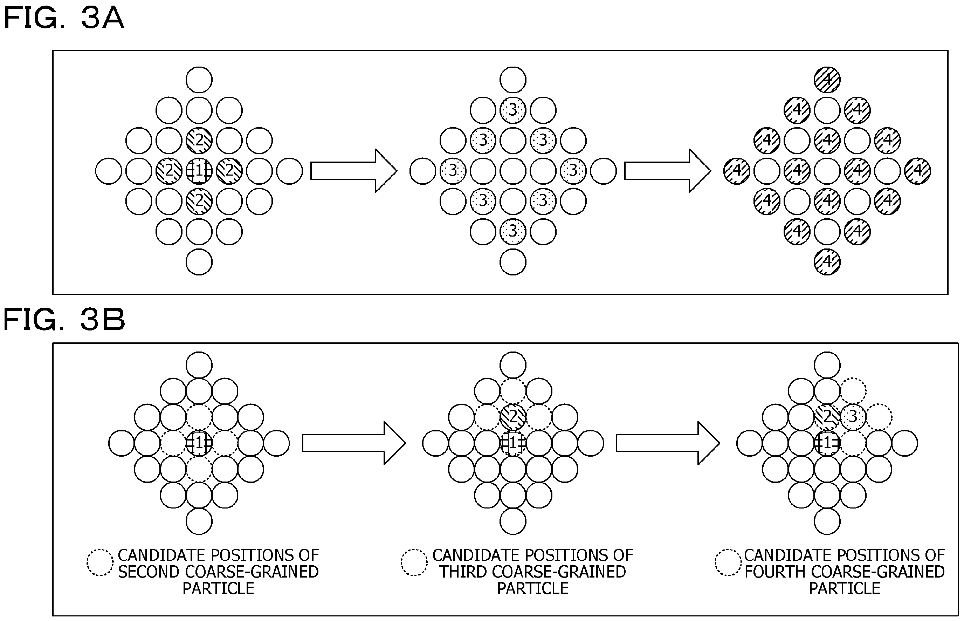

FIGs. 3A and 3B are diagrams illustrating a representation method with relative coordinates. Note that, in the example in FIGs. 3A and 3B, the space of the face-centered cubic is represented by a two-dimensional plane for simplification of description.

Among them, FIG. 3A illustrates, as a comparative example, a case where state variables are represented by absolute coordinates. As illustrated in FIG. 3A, when the position of the first coarse-grained particle included in the coarse-grained model is designated, the second coarse-grained particle has four different candidate positions adjacent to the first coarse-grained particle.

In addition, the third coarse-grained particle has eight different candidate positions adjacent to each of the candidate positions of the second coarse-grained particle. Furthermore, the fourth coarse-grained particle has 16 different candidate positions adjacent to each of the candidate positions of the third coarse-grained particle.

In this manner, when trying to specify the coordinates of each coarse-grained particle using a state variable represented by absolute coordinates, since the number of candidate positions of the coarse-grained particles is expanded as the number of coarse-grained particles included in the coarse-grained model becomes greater, the number of bits of the state variables increases.

Meanwhile, FIG. 3B illustrates a case where the state variables are represented by relative coordinates. As illustrated in FIG. 3B, when the position of the first coarse-grained particle included in the coarse-grained model is designated, the second coarse-grained particle has four different candidate positions adjacent to the first coarse-grained particle.

In addition, when the position of the second coarse-grained particle included in the coarse-grained model is designated, the third coarse-grained particle has three different candidate positions adjacent to the candidate position of the second coarse-grained particle. Furthermore, when the position of the third coarse-grained particle included in the coarse-grained model is designated, the fourth coarse-grained particle has three different candidate positions adjacent to the candidate position of the third coarse-grained particle.

In this manner, when the coordinates of each coarse-grained particle are specified using a state variable represented by relative coordinates, since the number of candidate positions of the i+1-th coarse-grained particle is unchanged regardless of the number of coarse-grained particles included in the coarse-grained model, the number of bits of the state variables is also not expanded.

That is, when the state variables are represented by relative coordinates as illustrated in FIG. 3B, there is an advantage that the number of bits of the state variables may be reduced.

<Specific Examples of State Variables>

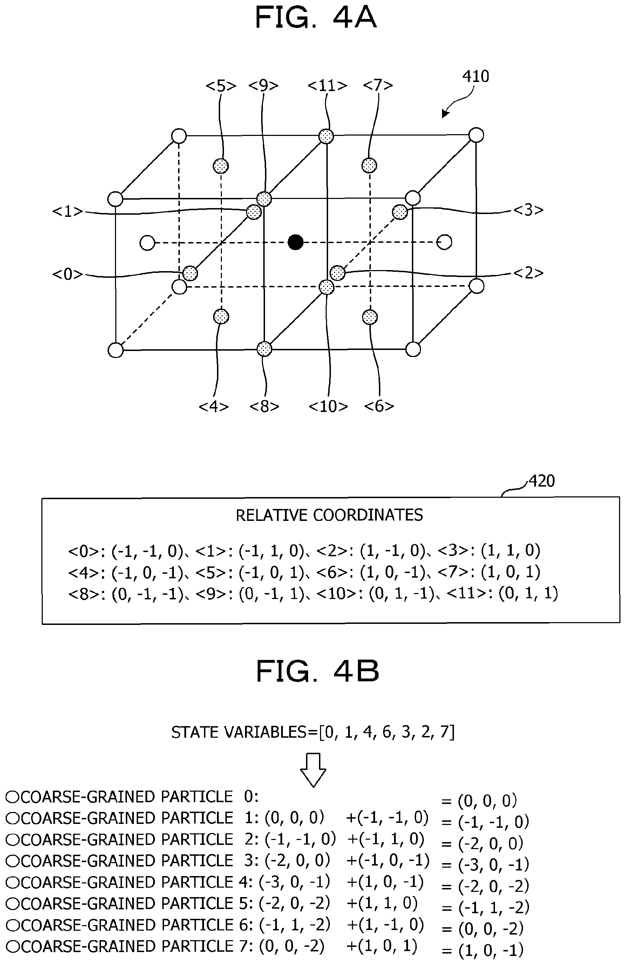

Next, a specific example of state variables represented by relative coordinates will be described. FIGs. 4A and 4B are diagrams illustrating a specific example of state variables.

As illustrated in FIG. 4A, it is assumed that the center position of a face-centered cubic (FCC) 410 is the origin (coordinates = (0, 0, 0)) and the i-th coarse-grained particle is located at the origin. In this case, the i+1-th coarse-grained particle has 12 different positions adjacent to the i-th coarse-grained particle (refer to <0> to <11>). Then, the relative coordinates of the 12 coarse-grained particles from the origin may be as follows:

Relative Coordinates of Position of <0>: (-1, -1, 0)
Relative Coordinates of Position of <1>: (-1, 1, 0)
Relative Coordinates of Position of < 2>: (1, -1, 0)
Relative Coordinates of Position of <3>: (1, 1, 0)
Relative Coordinates of Position of <4>: (-1, 0, -1)
Relative Coordinates of Position of <5>: (-1, 0, 1)
Relative Coordinates of Position of <6>: (1, 0, -1)
Relative Coordinates of Position of <7>: (0, -1, -1)
Relative Coordinates of Position of <8>: (0, -1, -1)
Relative Coordinates of Position of <9>: (0, -1, 1)
Relative Coordinates of Position of <10>: (0, 1, -1)
Relative Coordinates of Position of <11>: (0, 1, 1)

That is, the coordinates of the i+1-th coarse-grained particle adjacent to the i-th coarse-grained particle will be assigned to any one set of the above 12 different sets of relative coordinates (refer to the reference sign 420).

As an example, FIG. 4B illustrates an example in which the coordinates of each coarse-grained particle in a coarse-grained model including eight coarse-grained particles are calculated using state variables represented by relative coordinates. Specifically, when the coordinates of the zeroth coarse-grained particle are assumed as the origin (coordinates = (0, 0, 0)) and the state variables = [0, 1, 4, 6, 3, 2, 7] is assumed, examples of calculating may be as follows:

· the coordinates of the first coarse-grained particle = the coordinates of the zeroth coarse-grained particle + the relative coordinates of the position of <0>;
· the coordinates of the second coarse-grained particle = the coordinates of the first coarse-grained particle + the relative coordinates of the position of <1>;
· the coordinates of the third coarse-grained particle = the coordinates of the second coarse-grained particle + the relative coordinates of the position of <4>;
· the coordinates of the fourth coarse-grained particle = the coordinates of the third coarse-grained particle + the relative coordinates of the position of <6>;
· the coordinates of the fifth coarse-grained particle = the coordinates of the fourth coarse-grained particle + the relative coordinates of the position of <3>;
· the coordinates of the sixth coarse-grained particle = the coordinates of the fifth coarse-grained particle + the relative coordinates of the position of <2>; and
· the coordinates of the seventh coarse-grained particle = the coordinates of the sixth coarse-grained particle + the relative coordinates of the position of <7>.

<Details of Energy Computation Process>

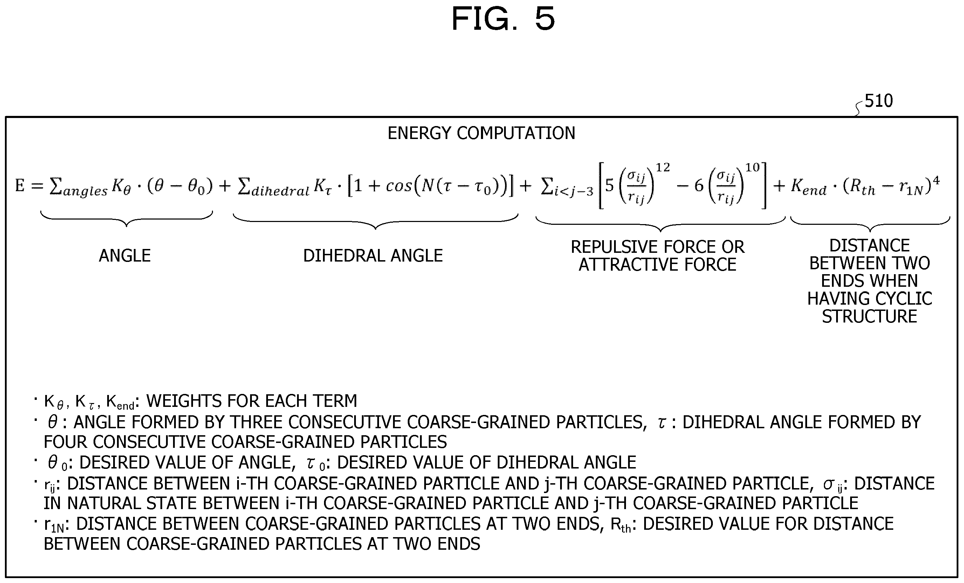

Next, details of an energy computation process based on the interaction between coarse-grained particles, which is executed by the stable structure search system according to the first embodiment when searching for a stable structure of the coarse-grained model, will be described. FIG. 5 is a diagram illustrating details of the energy computation process.

As illustrated in FIG. 5, the energy computation process (refer to the reference sign 510) executed when searching for a stable structure of the coarse-grained model includes the computation process for, as energy based on the interaction between the coarse-grained particles, · energy according to the angle between the coarse-grained particles, · energy according to the dihedral angle between the coarse-grained particles, · energy according to the repulsive force or the attractive force between the coarse-grained particles, and · energy according to the distance between the coarse-grained particles at two ends when having a cyclic structure, and is expressed by formula 1 below.
[Mathematical Formula 1]

$$E = \sum_{angles} K_\theta \cdot (\theta - \theta_0) + \sum_{dihedral} K_\tau \cdot \left[1 + cos\big(N(\tau - \tau_0)\big)\right]$$

$$+ \sum_{i<j-3} \left[5\left(\frac{\sigma_{ij}}{r_{ij}}\right)^{12} - 6\left(\frac{\sigma_{ij}}{r_{ij}}\right)^{10}\right] + K_{end} \cdot (R_{th} - r_{1N})^4$$

... Formula (1)

Note that, in above formula 1, Kθ, Kτ, and Kend denote weights for each term. In addition, in above formula 1, θ denotes the angle formed by three consecutive coarse-grained particles from the i-1-th to the i+1-th, and τ denotes the dihedral angle formed by four consecutive coarse-grained particles from the i-2-th to the i+1-th. In addition, θ0 denotes the desired value of the angle, and τ0 denotes the desired value of the dihedral angle. In addition, rij denotes the distance between the i-th coarse-grained particle and the j-th coarse-grained particle, and σij denotes the distance in a natural state between the i-th coarse-grained particle and the j-th coarse-grained particle. Furthermore, r1N denotes the distance between the coarse-grained particles at the two ends, and Rth denotes the desired value of the distance between the coarse-grained particles at the two ends.

Here, in executing the above energy computation process, the stable structure search system according to the first embodiment treats the difference in energy values caused along with moving the position of each coarse-grained particle, as a computation object. This enables the stable structure search system according to the first embodiment to reduce the amount of computation for the energy computation process.

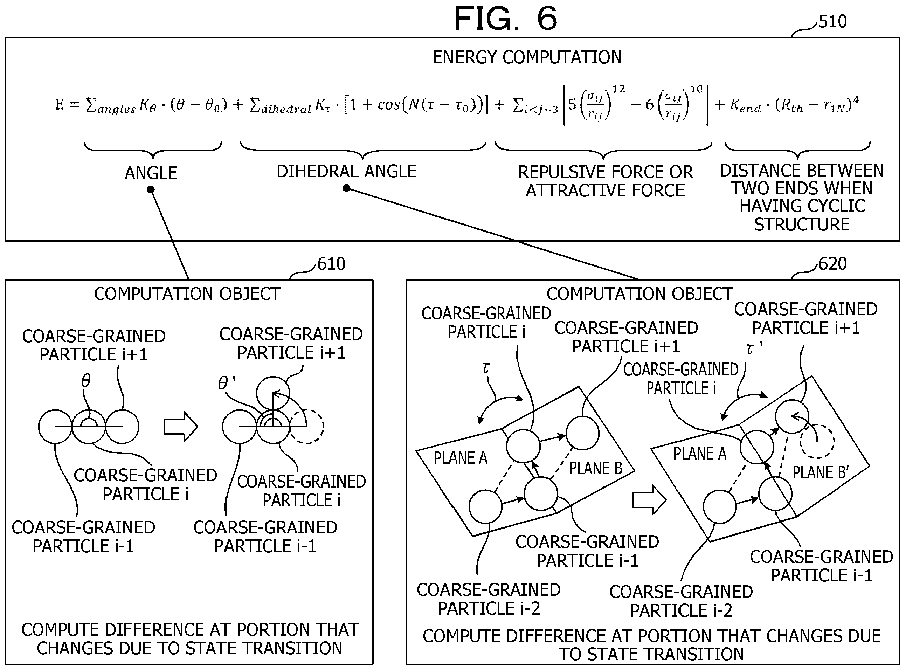

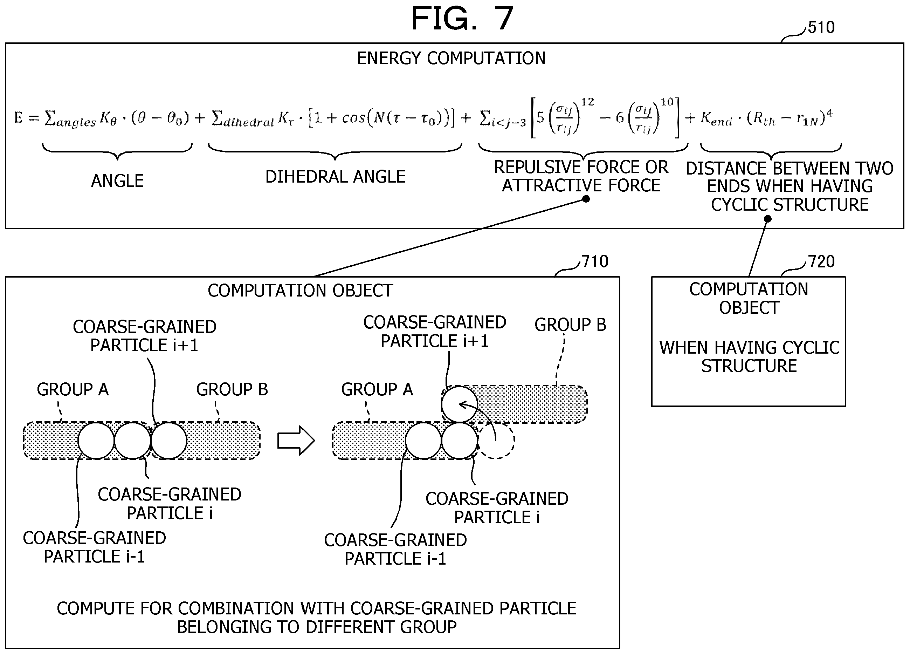

FIGs. 6 and 7 are first and second diagrams illustrating details of each term of the energy computation process. As indicated by the reference sign 610 in FIG. 6, when computing the energy according to the angle between the coarse-grained particles, the stable structure search system according to the first embodiment treats the difference in energy values caused along with moving the positions of the coarse-grained particles, as a computation object.

Specifically, when the state of the coarse-grained model has transitioned due to the movement of the i+1-th coarse-grained particle relative to the i-th coarse-grained particle, the energy according to the difference (= θ-θ') between · the angle θ formed between the i-1-th coarse-grained particle, the i-th coarse-grained particle, and the i+1-th coarse-grained particle before the i+1-th coarse-grained particle moves (the angle θ around the i-th coarse-grained particle before the i+1-th coarse-grained particle moves), and · the angle θ' formed between the i-1-th coarse-grained particle, the i-th coarse-grained particle, and the i+1-th coarse-grained particle after the i+1-th coarse-grained particle moves (the angle θ' around the i-th coarse-grained particle after the i+1-th coarse-grained particle moves) is computed as the energy according to the angle between the coarse-grained particles. In other words, the angle around the i-1-th coarse-grained particle, the angle around the i-2-th coarse-grained particle,..., and the like do not change between before and after the i+1-th coarse-grained particle moves and are therefore not included in the computation of the energy according to the angle between the coarse-grained particles. In addition, the i+2-th and subsequent coarse-grained particles each move in parallel while maintaining the angles when the i+1-th coarse-grained particle moves. That is, the angle around the i+1-th coarse-grained particle, the angle around the i+2-th coarse-grained particle,..., and the like do not change between before and after the i+1-th coarse-grained particle moves and are therefore not included in the computation of the energy according to the angle between the coarse-grained particles.

Similarly, as indicated by the reference sign 620 in FIG. 6, when computing the energy according to the dihedral angle between the coarse-grained particles, the stable structure search system according to the first embodiment treats the difference in energy values caused along with moving the positions of the coarse-grained particles, as a computation object.

Specifically, when the state of the coarse-grained model has transitioned due to the movement of the i+1-th coarse-grained particle relative to the i-th coarse-grained particle, the energy according to the difference (= τ-τ') between · the dihedral angle τ formed between a plane A formed by the i-2-th to i-th coarse-grained particles and a plane B formed by the i-1-th to i+1-th coarse-grained particles before the i+1-th coarse-grained particle moves, and · the dihedral angle τ' formed between the plane A formed by the i-2-th to i-th coarse-grained particles and a plane B' formed by the i-1-th to i+1-th coarse-grained particles after the i+1-th coarse-grained particle moves is computed as the energy according to the dihedral angle between the coarse-grained particles. In other words, the dihedral angle formed between the plane formed by the i-3-th to i-1-th coarse-grained particles and the plane formed by the i-2-th to i-th coarse-grained particles, and the like do not change between before and after the i+1-th coarse-grained particle moves. For this reason, these dihedral angles are not included in the computation of the energy according to the dihedral angle between the coarse-grained particles. In addition, the i+2-th and subsequent coarse-grained particles each move in parallel while maintaining the angles when the i+1-th coarse-grained particle moves. That is, the dihedral angle formed between the plane formed by the i-1-th to i+1-th coarse-grained particles and the plane formed by the i-th to i+2-th coarse-grained particles, and the like do not change between before and after the i+1-th coarse-grained particle moves. For this reason, these dihedral angles are not included in the computation of the energy according to the dihedral angle between the coarse-grained particles.

In addition, as indicated by the reference sign 710 in FIG. 7, when computing the energy according to the repulsive force or the attractive force between coarse-grained particles, the stable structure search system according to the first embodiment treats all combinations constituted among coarse-grained particles belonging to different groups from each other, as computation objects. In other words, the repulsive force or the attractive force between the coarse-grained particles belonging to the same group is not included in the energy computation.

Specifically, when the state of the coarse-grained model has transitioned due to the movement of the i+1-th coarse-grained particle relative to the i-th coarse-grained particle, the energy according to the difference between · the repulsive force or the attractive force computed for all combinations among respective coarse-grained particles separately belonging to a group A (the group to which each of i-th and preceding coarse-grained particles belongs) and a group B (the group to which each of i+1-th and subsequent coarse-grained particles belongs) before the i+1-th coarse-grained particle moves, and · the repulsive force or the attractive force computed for all combinations among respective coarse-grained particle separately belonging to the group A (the group to which each of i-th and preceding coarse-grained particle belongs) and the group B (the group to which each of i+1-th and subsequent coarse-grained particles belongs) after the i+1-th coarse-grained particle moves are computed as the energy according to the repulsive force or the attractive force between the coarse-grained particles. In other words, the energy according to the repulsive force or the attractive force between the coarse-grained particles belonging to the group A does not change between before and after the i+1-th coarse-grained particle moves and is therefore not included in the computation of the energy according to the repulsive force or the attractive force between the coarse-grained particles. In addition, since each coarse-grained particle belonging to the group B moves in parallel while maintaining the distance when the i+1-th coarse-grained particle moves, the repulsive force or the attractive force between the coarse-grained particles belonging to the group B does not change between before and after the i+1-th coarse-grained particle moves. For this reason, this repulsive force or attractive force is not included in the computation of the energy according to the repulsive force or the attractive force between the coarse-grained particles.

Note that, as indicated by the reference sign 720 in FIG. 7, in a case where the coarse-grained model has a cyclic structure, when the state of the coarse-grained model transitions due to the movement of the i+1-th coarse-grained particle relative to the i-th coarse-grained particle, the distance between the coarse-grained particles at the two ends regularly changes.

For this reason, the energy according to the distance between the coarse-grained particles at the two ends when having a cyclic structure is normally computed every time the i+1-th coarse-grained particle moves (is not excluded from the computation object when the coarse-grained model has a cyclic structure).

<System Configuration of Stable Structure Search System and Functional Configuration of Each Device>

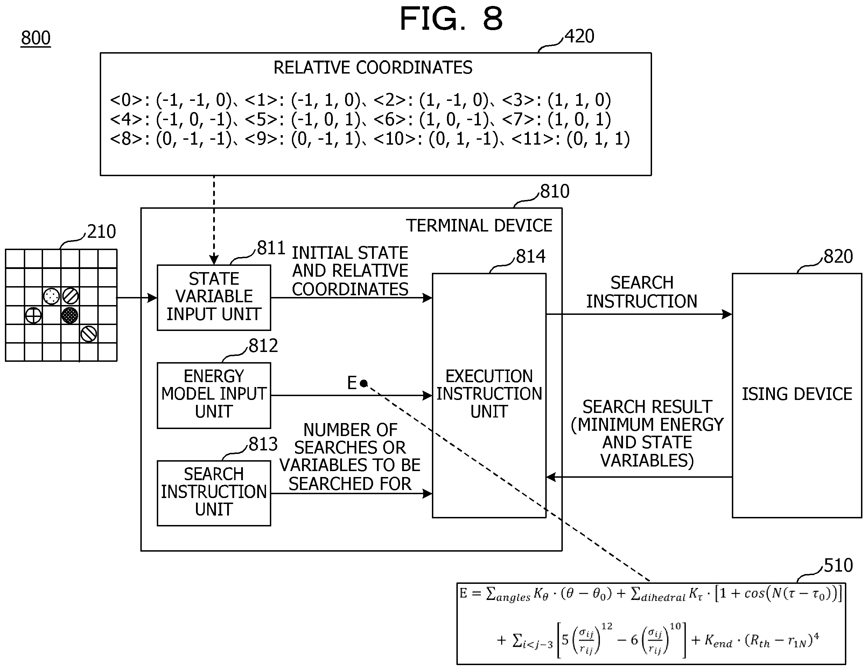

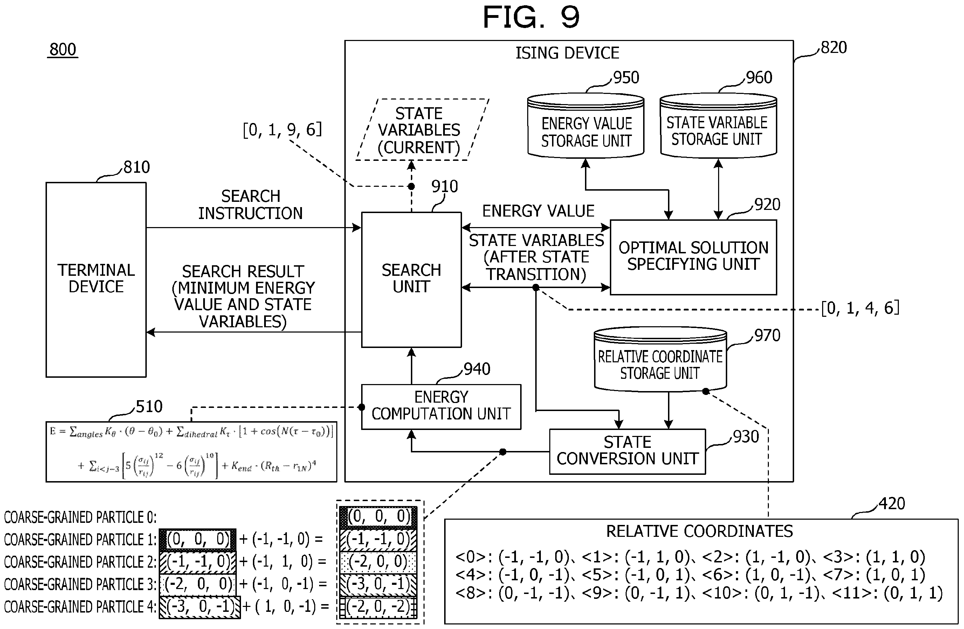

Next, a system configuration of the stable structure search system according to the first embodiment and a functional configuration of each device will be described with reference to FIGs. 8 and 9. FIG. 8 is a diagram illustrating an example of the system configuration of the stable structure search system and the functional configuration of a terminal device. In addition, FIG. 9 is a diagram illustrating an example of the system configuration of the stable structure search system and the functional configuration of an Ising device.

System Configuration of Stable Structure Search System and Functional Configuration of Terminal Device

As illustrated in FIG. 8, a stable structure search system 800 according to the first embodiment includes a terminal device 810 and an Ising device 820.

A stable structure search instruction program is installed in the terminal device 810, and when the program is executed, the terminal device 810 functions as a state variable input unit 811, an energy model input unit 812, a search instruction unit 813, and an execution instruction unit 814.

The state variable input unit 811 accepts input of the coarse-grained model in an initial state (reference sign 210), which is a search object for a stable structure. Note that the state variable input unit 811 specifies the coarse-grained model (reference sign 210) by accepting settings for the number of amino acid residues N (N is an integer equal to or greater than two) and the amino acid residue sequence. In addition, the state variable input unit 811 assigns the initial state of the coarse-grained model (reference sign 210) by randomly designating N-1 state variables.

The state variable input unit 811 also accepts input of relative coordinates (reference sign 420) for representing the state variables. Note that the execution instruction unit 814 is notified of the coarse-grained model in the initial state (reference sign 210) and the relative coordinates (reference sign 420) whose input has been accepted by the state variable input unit 811.

The energy model input unit 812 accepts input of a computation formula (reference sign 510) used when executing the energy computation process based on the interaction between the coarse-grained particles. Note that the execution instruction unit 814 is notified of the computation formula (reference sign 510) whose input has been accepted by the energy model input unit 812.

The search instruction unit 813 accepts input of the number of searches when searching for a stable structure or variables to be searched for, for the coarse-grained model (reference sign 210). The here mentioned number of searches or variables to be searched for are equivalent to the number of updates of the state variables in the Ising device 820. Note that the execution instruction unit 814 is notified of the number of searches or the variables to be searched for, of which input has been accepted by the search instruction unit 813.

The execution instruction unit 814 instructs the Ising device 820 to search for a stable structure, based on each piece of information notified by the state variable input unit 811, the energy model input unit 812, and the search instruction unit 813. Note that, by transmitting · the search object: the coarse-grained model (reference sign 210), · the initial state of the coarse-grained model: the state variables designated at random, · the state variable representation method: relative coordinates (reference sign 420), · the energy computation method during the search process: the computation formula (reference sign 510), and · the number of updates of the state variables during the search process: the number of searches or the variables to be searched for, the execution instruction unit 814 issues a search instruction to the Ising device 820.

In addition, by issuing the search instruction to the Ising device 820, the execution instruction unit 814 receives a search result from the Ising device 820. The search result received by the execution instruction unit 814 includes the computation result for the minimum energy and the state variables at that time. This allows the execution instruction unit 814 to output a stable structure for the coarse-grained model (reference sign 210) and the energy based on the interaction between the coarse-grained particles of the coarse-grained model (reference sign 220) in the stable structure.

(2) System Configuration of Stable Structure Search System and Functional Configuration of Ising Device

Subsequently, a functional configuration of the Ising device 820 will be described with reference to FIG. 9 (since the system configuration of the stable structure search system 800 has already been described with reference to FIG. 8, description thereof will be omitted here).

The stable structure search program is installed in the Ising device 820, and when the program is executed, the Ising device 820 functions as a search unit 910, an optimal solution specifying unit 920, a state conversion unit 930, and an energy computation unit 940.

The search unit 910 searches for a stable structure for the coarse-grained model (reference sign 210) based on the search instruction from the terminal device 810 and transmits the search result to the terminal device 810. Specifically, the search unit 910 updates (alters) the state variables of the coarse-grained model (reference sign 210) within the range of the instructed number of searches (or variables to be searched for). In addition, the search unit 910 determines whether or not the transition is permitted, based on the energy value calculated every time a state variable is updated and the state of the coarse-grained model is transitioned. In addition, the search unit 910 transmits the minimum energy value specified from among the energy values determined that the transition is permitted, and the state variables at that time, to the terminal device 810 as a search result.

The example in FIG. 9 illustrates how the search unit 910 notifies the optimal solution specifying unit 920 of the energy values computed by the energy computation unit 940 for the current state variables ([0, 1, 9, 6]). The example in FIG. 9 also illustrates how the search unit 910 updates the state variables from [0, 1, 9, 6] to [0, 1, 4, 6] and notifies the optimal solution specifying unit 920 and the state conversion unit 930 of the updated state variables.

The optimal solution specifying unit 920 is an example of a specifying unit. The optimal solution specifying unit 920 compares the energy value stored in an energy value storage unit 950 every time the energy value is notified by the search unit 910 and, when the notified energy value is smaller, stores the notified energy value in the energy value storage unit 950. This allows the optimal solution specifying unit 920 to specify the minimum energy value.

In addition, the optimal solution specifying unit 920 holds the updated state variables every time the updated state variables are notified by the search unit 910 and, when the corresponding energy value is notified by the search unit 910 and the corresponding energy value is stored in the energy value storage unit 950, stores the held state variables in a state variable storage unit 960. This allows the optimal solution specifying unit 920 to specify the state variables corresponding to the minimum energy value.

The state conversion unit 930 is an example of a calculation unit and, every time the updated state variables are notified by the search unit 910, refers to a relative coordinate storage unit 970 to calculate the coordinates of each coarse-grained particle of the coarse-grained model after the state transition. Note that it is assumed that the relative coordinates transmitted along with the search instruction from the terminal device 810 have been stored in the relative coordinate storage unit 970.

The example in FIG. 9 illustrates how the state conversion unit 930 calculates the coordinates of each coarse-grained particle (a coarse-grained particle 0 to a coarse-grained particle 4) as follows, based on the relative coordinates, when notified of [0, 1, 4, 6] by the search unit 910 as the updated state variables: · coarse-grained particle 0: (0, 0, 0), · coarse-grained particle 1: (0, 0, 0) + (-1, -1, 0) = (-1, -1, 0), · coarse-grained particle 2: (-1, -1, 0) + (-1, 1, 0) = (-2, 0, 0), · coarse-grained particle 3: (-2, 0, 0) + (-1, 0, -1) = (-3, 0, -1), · coarse-grained particle 4: (-3, 0, -1) + (1, 0, -1) = (-2, 0, -2).

Note that the energy computation unit 940 is notified of the coordinates of each coarse-grained particle of the coarse-grained model after the state transition calculated by the state conversion unit 930.

The energy computation unit 940 executes the energy computation process every time the coordinates of each coarse-grained particle of the coarse-grained model after the state transition are notified by the state conversion unit 930. Specifically, the energy computation unit 940 computes the energy values for the computation objects described with reference to FIGs. 6 and 7, based on the computation formula (reference sign 510) transmitted along with the search instruction from the terminal device 810. Note that the search unit 910 is notified of the energy values computed by the energy computation unit 940, and by handling the updated state variables ([0, 1, 4, 6]) as the state variables before update, the search unit 910 performs the following search.

<Hardware Configuration of Terminal Device>

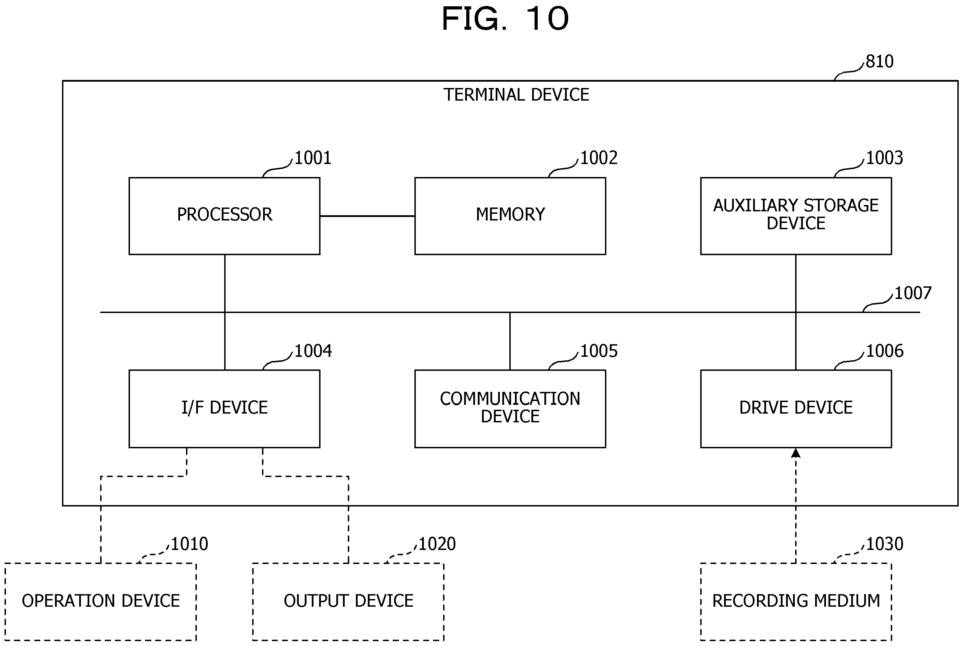

Next, a hardware configuration of the terminal device 810 will be described. FIG. 10 is a diagram illustrating an example of the hardware configuration of the terminal device.

As illustrated in FIG. 10, the terminal device 810 includes a processor 1001, a memory 1002, an auxiliary storage device 1003, an interface (I/F) device 1004, a communication device 1005, and a drive device 1006. Note that the processor 1001, the auxiliary storage device 1003, the I/F device 1004, the communication device 1005, and the drive device 1006 of the terminal device 810 are coupled to each other via a bus 1007.

The processor 1001 includes various arithmetic devices such as a central processing unit (CPU) and a graphics processing unit (GPU). The processor 1001 reads various programs (such as the stable structure search instruction program as an example) into the memory 1002 to execute the read various programs.

The memory 1002 includes a main storage device such as a read only memory (ROM) or a random access memory (RAM). The processor 1001 and the memory 1002 form a so-called computer. The processor 1001 executes various programs read into the memory 1002 to cause the computer to implement the above various functions.

The auxiliary storage device 1003 stores various programs and various sorts of information used when the various programs are executed by the processor 1001.

The I/F device 1004 is a coupling device that couples an operation device 1010 and an output device 1020, which are examples of external devices, with the terminal device 810.

The communication device 1005 is a communication device for communicating with the Ising device 820 via a network.

The drive device 1006 is a device for setting a recording medium 1030. The recording medium 1030 mentioned here includes a medium that optically, electrically, or magnetically records information, such as a compact disc read only memory (CD-ROM), a flexible disk, or a magneto-optical disk. In addition, the recording medium 1030 may include a semiconductor memory or the like that electrically records information, such as a ROM or a flash memory.

Note that various programs installed in the auxiliary storage device 1003 are installed, for example, by setting the distributed recording medium 1030 in the drive device 1006 and reading various programs recorded in the recording medium 1030. Alternatively, various programs installed in the auxiliary storage device 1003 may be installed by being downloaded from a network via the communication device 1005.

Note that only the hardware configuration of the terminal device 810 has been described here, and the hardware configuration of the Ising device 820 has not been described. However, the hardware configuration of the Ising device 820 may be similar to the hardware configuration of the terminal device 810, for example. Alternatively, the hardware configuration of the Ising device 820 may be similar to the hardware configuration of a so-called quantum computer.

<Flow of Stable Structure Search Process>

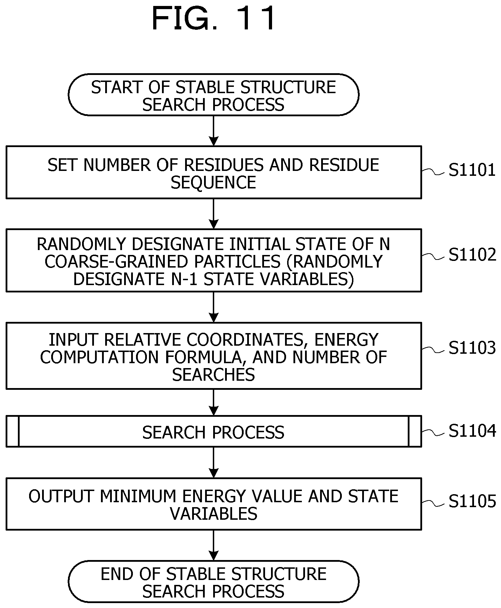

Next, a flow of the stable structure search process by the stable structure search system 800 will be described. FIG. 11 is a flowchart illustrating a flow of the stable structure search process.

In step S1101, the terminal device 810 specifies a coarse-grained model as a search object for a stable structure, by accepting settings for the number of amino acid residues N (N is an integer equal to or greater than two) and the amino acid residue sequence.

In step S1102, the terminal device 810 randomly designates the initial state for the coarse-grained model with the N coarse-grained particles. Specifically, the terminal device 810 randomly designates N-1 state variables.

In step S1103, the terminal device 810 accepts input of relative coordinates for representing the state variables. The terminal device 810 also accepts input of the computation formula used for the energy computation process during the search process. In addition, the terminal device 810 accepts the number of searches or the variables to be searched for, as the number of updates of the state variables during the search process. Furthermore, the terminal device 810 transmits the search instruction to the Ising device 820 to cause the Ising device 820 to search for a stable structure of the coarse-grained model, based on each piece of the accepted information.

In step S1104, the Ising device 820 executes the search process. Note that details of the search process by the Ising device 820 will be described later.

In step S1105, the terminal device 810 receives the search result from the Ising device 820 and outputs the computation result for the minimum energy value for the coarse-grained model as a search object, and the state variables at that time.

<Flow of Search Process>

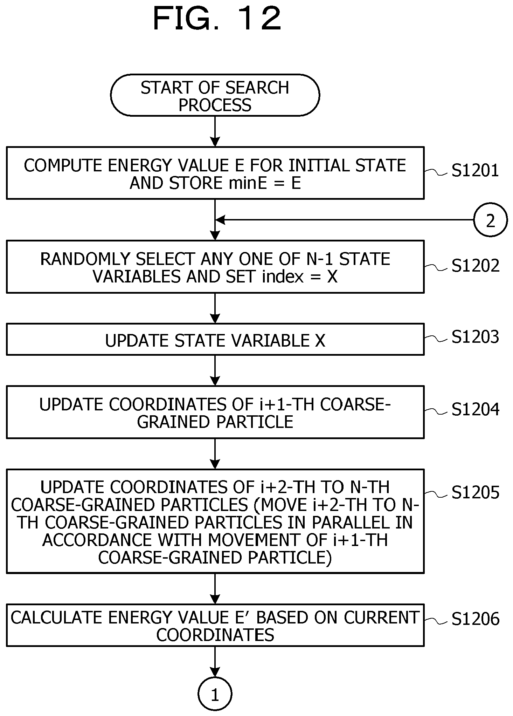

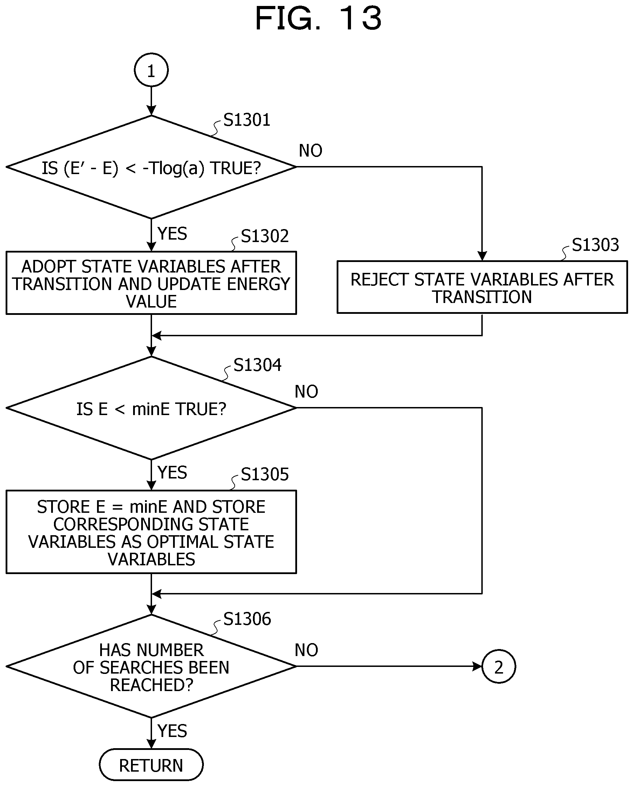

Next, details of the search process (step S1104) by the Ising device 820 will be described with reference to FIGs. 12 and 13. FIGs. 12 and 13 are first and second flowcharts illustrating details of the search process.

In step S1201 in FIG. 12, the Ising device 820 computes the energy value for the coarse-grained model (reference sign 210) in the initial state and stores the computed energy value as the minimum energy value (minE) at the current time point.

In step S1202, the Ising device 820 randomly selects any one of the N-1 state variables that specify the coordinates of the N coarse-grained particles of the coarse-grained model, and sets index = X (X = any integer from 0 to N-2).

In step S1203, the Ising device 820 updates the state variable X. Note that, here, the coarse-grained particle that moves due to updating the state variable X is assumed to be the i+1-th coarse-grained particle.

In step S1204, the Ising device 820 calculates the coordinates of the i+1-th coarse-grained particle. Specifically, the Ising device 820 calculates the coordinates of the i+1-th coarse-grained particle by adding the relative coordinates corresponding to the state variable X to the coordinates of the i-th coarse-grained particle.

In step S1205, the Ising device 820 updates the coordinates of the i+2-th to N-th coarse-grained particles. Specifically, the Ising device 820 updates the coordinates of the i+2-th to N-th coarse-grained particles, by moving the i+2-th to N-th coarse-grained particles in parallel in accordance with the movement of the i+1-th coarse-grained particle.

In step S1206, the Ising device 820 computes the energy value E' based on the coordinates of each coarse-grained particle of the coarse-grained model after transition, of which the state has transitioned due to updating the state variable X.

Subsequently, in step S1301 in FIG. 13, the Ising device 820 calculates the difference between the energy value E stored as the minimum energy value (minE) at the current time point and the energy value E' computed in step S1206. In addition, the Ising device 820 determines whether or not the calculated difference is less than a predetermined thermal noise (-Tlog(a) in the example in FIG. 13). Note that a denotes a uniform random number and is a value that satisfies a ∈ [0, 1].

When it is determined in step S1301 that the calculated difference is less than the predetermined thermal noise (-Tlog(a)) (in the case of YES in step S1301), the process proceeds to step S1302.

In step S1302, the Ising device 820 determines that transition is permitted and adopts the updated state variables, while updating the energy value (replacing the energy value E with the energy value E').

On the other hand, when it is determined in step S1301 that the calculated difference is equal to or greater than the predetermined thermal noise (-Tlog(a)) (in the case of NO in step S1301), the process proceeds to step S1303.

In step S1303, the Ising device 820 determines that transition is not permitted and rejects the updated state variables.

In step S1304, the Ising device 820 determines whether or not the energy value E is smaller than the minimum energy value (minE). When it is determined in step S1304 that the energy value E is smaller than the minimum energy value (minE) (in the case of YES in step S1304), the process proceeds to step S1305.

In step S1305, the Ising device 820 stores the energy value E as the minimum energy value (minE) at the current time point. The Ising device 820 also stores the corresponding state variables as the optimal state variables at the current time point.

On the other hand, when it is determined in step S1304 that the energy value E is equal to or greater than the minimum energy value (minE) (in the case of NO in step S1304), the process proceeds to step S1306.

In step S1306, the Ising device 820 determines whether or not the number of updates of the state variables has reached the number of searches (or the variables to be searched for) transmitted along with the search instruction. When it is determined in step S1306 that the number of searches (or the variables to be searched for) has not been reached (in the case of NO in step S1306), the process returns to step S1202 in FIG. 12.

On the other hand, when it is determined in step S1306 that the number of searches (or the variables to be searched for) has been reached (in the case of YES in step S1306), the minimum energy value (minE) and the optimal state variables are transmitted to the terminal device 810 as a search result. Thereafter, the process returns to step S1105.

<Effect of Reduction in Amount of Computation>

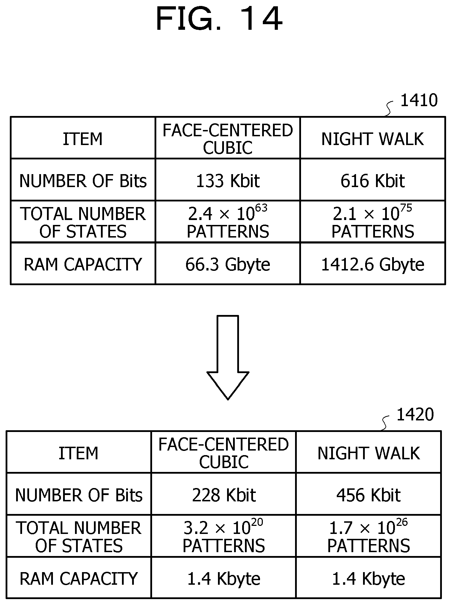

Next, an effect of reduction in the amount of computation during the stable structure search process by the stable structure search system 800 according to the first embodiment will be described. FIG. 14 is a diagram illustrating an example of the effect of reduction in the amount of computation.

In this, the reference sign 1410 indicates, as a comparative example, the amount of computation when searching for a stable structure by · using state variables represented by absolute coordinates, and · computing the energy without limiting the computation objects when the number of coarse-grained particles N = 20 is employed.

Meanwhile, the reference sign 1420 indicates the amount of computation when the stable structure search system 800 according to the first embodiment searches for a stable structure by · using state variables represented by relative coordinates, and · treating the difference in energy caused along with moving the position of the coarse-grained particle, as the computation object, when the number of coarse-grained particles N = 20 is employed.

Note that, among the respective items relating to the amount of computation indicated by the reference signs 1410 and 1420, the "number of bits" denotes the number of bits involved in representing the coordinates of each coarse-grained particle. In addition, the "total number of states" denotes the number of combinations of coordinates that each coarse-grained particle included in the coarse-grained model can take. In addition, the "RAM capacity" denotes the memory capacity involved during the energy computation process.

In addition, the amount of computation for each item is indicated for each of the case where the lattice space is a face-centered cubic and the case where the lattice space is a night walk.

As is clear from the comparison between the reference signs 1410 and 1420, the stable structure search system 800 according to the first embodiment may be enabled to greatly reduce each amount of computation during the stable structure search process. In other words, the stable structure search system 800 according to the first embodiment may be enabled to greatly raise the number of coarse-grained particles N of the coarse-grained model as a search object, under the computer power in the current state.

As is clear from the above description, in a coarse-grained model in which N coarse-grained particles arranged in a shape of a row are arranged in a lattice space, the stable structure search system 800 according to the first embodiment calculates the coordinates in the lattice space of the i+1-th coarse-grained particle in the row, by using · the coordinates in the lattice space of the i-th coarse-grained particle in the row, and · state variables represented by relative coordinates in the lattice space of the i-th coarse-grained particle and the i+1-th coarse-grained particle. In addition, the stable structure search system 800 according to the first embodiment repeatedly executes the process of computing the value of energy of the coarse-grained model, based on the coordinates in the lattice space of each of the N coarse-grained particles under the limited computation objects, every time the state variables are altered.

This may enable the stable structure search system according to the first embodiment to specify the state variables with the minimum energy and reduce the amount of computation when searching for a stable structure of the coarse-grained model.

[Second Embodiment]

In the above first embodiment, the case of randomly selecting and updating any one of the N-1 state variables during the search process has been described, but the state variables may be selected in a manner different than the selecting of the state variables at random. For example, the selection and update may be made in a predetermined order, such as sequentially selecting from the first state variable to the N-1-th state variable.

Note that, in the case of the face-centered cubic, 12 different values are included in the values of the updated state variable, and it is assumed to be designated at random to which value the state variable is to be updated.

In addition, in the above first embodiment, it has been assumed that the weight of each term, such as $K\theta$, $K\tau$, and Kend, is fixed in the computation formula (reference sign 510) used for the energy computation process. However, the weight of each term may be altered, for example, according to the type of amino acid.

In addition, in the above first embodiment, the stable structure search system 800 has been described as including the terminal device 810 and the Ising device 820. However, the terminal device 810 and the Ising device 820 may form an integrated device. In this case, it is assumed that the stable structure search program includes the stable structure search instruction program, and each functional unit of the terminal device 810 described with reference to FIG. 8 and each functional unit of the Ising device 820 described with reference to FIG. 9 are implemented in the Ising device 820.

Note that the present embodiments are not limited to the configurations described here and may include, for example, combinations of the configurations or the like described in the above embodiments and other elements. These points may be altered without departing from the spirit of the embodiments and may be appropriately defined according to application modes thereof.

**Claims**

1.  A stable structure search system comprising:

    a calculation unit that, in a model in which N particles (N is an integer equal to or greater than two) arranged in a shape of a row are arranged in a lattice space, calculates coordinates in the lattice space of an i+1-th particle in the row, by using the coordinates in the lattice space of an i-th particle in the row, and state variables represented by relative coordinates in the lattice space of the i-th particle and the i+1-th particle;
    a computation unit that computes a value of energy of the model, based on the coordinates in the lattice space of each of the N particles, every time the state variables are altered; and
    a specifying unit that specifies the state variables with which the value of the energy has a local minimum value.

2.  The stable structure search system according to claim 1, further comprising a search unit that alters any one variable among N-1 variables included in the state variables.

3.  The stable structure search system according to claim 1, wherein the relative coordinates are the coordinates of the i+1-th particle when the i-th particle is arranged at an origin in the lattice space.

4.  The stable structure search system according to claim 3, wherein the calculation unit calculates the coordinates of the i+1-th particle when the state variables are altered, based on the altered state variables.

5.  The stable structure search system according to claim 4, wherein the calculation unit calculates the coordinates of each of an i+2-th particle to an N-th particle, by assuming that each of the i+2-th particle to the N-th particle moves in parallel along with a movement of the i+1-th particle.

6.  The stable structure search system according to claim 2, wherein the computation unit computes the value of the energy based on an interaction between the N particles, based on the coordinates in the lattice space of each of the N particles.

7.  The stable structure search system according to claim 6, wherein the energy includes at least any one of: an energy according to angles between the N particles, an energy according to dihedral angles between the N particles, an energy according to a repulsive force or an attractive force between the N particles, and an energy according to a distance between the particles at two ends when the N particles have a cyclic structure.

8.  The stable structure search system according to claim 5, wherein the computation unit computes a difference between the energy after the state variables are altered and the energy before the state variables are altered.

9.  The stable structure search system according to claim 6, wherein the search unit determines that alteration of the state variables is permitted when the difference computed by the computation unit satisfies a predetermined condition.

10. The stable structure search system according to claim 9, wherein the specifying unit specifies, from among the state variables altered within a range of a predetermined number of updates, the state variables when determined by the search unit to be alterable and with which the energy that corresponds to the state variables has the local minimum value.

11. A stable structure search method implemented by a computer, the method comprising:

    calculating, in a model in which N particles (N is an integer equal to or greater than two) arranged in a shape of a row are arranged in a lattice space, coordinates in the lattice space of an i+1-th particle in the row, by using

the coordinates in the lattice space of an i-th particle in the row, and state variables represented by relative coordinates in the lattice space of the i-th particle and the i+1-th particle;

computing a value of energy of the model, based on the coordinates in the lattice space of each of the N particles, every time the state variables are altered; and

specifying the state variables with which the value of the energy has a local minimum value.

**12.** A stable structure search program comprising instructions which, when executed by a computer, cause the computer to perform processing comprising:

calculating, in a model in which N particles (N is an integer equal to or greater than two) arranged in a shape of a row are arranged in a lattice space, coordinates in the lattice space of an i+1-th particle in the row, by using the coordinates in the lattice space of an i-th particle in the row, and state variables represented by relative coordinates in the lattice space of the i-th particle and the i+1-th particle;

computing a value of energy of the model, based on the coordinates in the lattice space of each of the N particles, every time the state variables are altered; and

specifying the state variables with which the value of the energy has a local minimum value.

## FIG. 1

# FIG. 2

COARSE-GRAINED MODEL IN INITIAL STATE (210)

SEARCH FOR STABLE STRUCTURE

COARSE-GRAINED MODEL HAVING STABLE STRUCTURE (220)

SEARCH FOR COARSE-GRAINED MODEL HAVING STABLE STRUCTURE BY SPECIFYING MINIMUM ENERGY VALUE
→USE MARKOV-CHAIN MONTE CARLO AND PARALLEL TEMPERING

+

· CALCULATE COORDINATES OF i+1-TH COARSE-GRAINED PARTICLE USING STATE VARIABLE REPRESENTED BY COORDINATES RELATIVE TO COORDINATES OF i-TH COARSE-GRAINED PARTICLE

· COMPUTE ENERGY BASED ON INTERACTION BETWEEN COARSE-GRAINED PARTICLES, BASED ON COORDINATES OF EACH COARSE-GRAINED PARTICLE CALCULATED USING STATE VARIABLE

EP 4 293 674 A1

FIG. 3A

FIG. 3B

CANDIDATE POSITIONS OF SECOND COARSE-GRAINED PARTICLE

CANDIDATE POSITIONS OF THIRD COARSE-GRAINED PARTICLE

CANDIDATE POSITIONS OF FOURTH COARSE-GRAINED PARTICLE

# FIG. 4A

420

RELATIVE COORDINATES

<0>: (-1, -1, 0)、 <1>: (-1, 1, 0)、 <2>: (1, -1, 0)、 <3>: (1, 1, 0)
<4>: (-1, 0, -1)、 <5>: (-1, 0, 1)、 <6>: (1, 0, -1)、 <7>: (1, 0, 1)
<8>: (0, -1, -1)、 <9>: (0, -1, 1)、 <10>: (0, 1, -1)、 <11>: (0, 1, 1)

# FIG. 4B

STATE VARIABLES=[0, 1, 4, 6, 3, 2, 7]

⬇

○COARSE-GRAINED PARTICLE  0:                                      = (0, 0, 0)
○COARSE-GRAINED PARTICLE  1: (0, 0, 0)    +(-1, -1, 0)   = (-1, -1, 0)
○COARSE-GRAINED PARTICLE  2: (-1, -1, 0)  +(-1, 1, 0)    = (-2, 0, 0)
○COARSE-GRAINED PARTICLE  3: (-2, 0, 0)   +(-1, 0, -1)   = (-3, 0, -1)
○COARSE-GRAINED PARTICLE  4: (-3, 0, -1)  +(1, 0, -1)    = (-2, 0, -2)
○COARSE-GRAINED PARTICLE  5: (-2, 0, -2)  +(1, 1, 0)     = (-1, 1, -2)
○COARSE-GRAINED PARTICLE  6: (-1, 1, -2)  +(1, -1, 0)    = (0, 0, -2)
○COARSE-GRAINED PARTICLE  7: (0, 0, -2)   +(1, 0, 1)     = (1, 0, -1)

## FIG. 5

510

ENERGY COMPUTATION

$$E = \sum_{angles} K_\theta \cdot (\theta - \theta_0) + \sum_{dihedral} K_\tau \cdot \left[1 + cos\left(N(\tau - \tau_0)\right)\right] + \sum_{i<j-3} \left[5\left(\frac{\sigma_{ij}}{r_{ij}}\right)^{12} - 6\left(\frac{\sigma_{ij}}{r_{ij}}\right)^{10}\right] + K_{end} \cdot (R_{th} - r_{1N})^4$$

| ANGLE | DIHEDRAL ANGLE | REPULSIVE FORCE OR ATTRACTIVE FORCE | DISTANCE BETWEEN TWO ENDS WHEN HAVING CYCLIC STRUCTURE |

- $K_\theta$, $K_\tau$, $K_{end}$: WEIGHTS FOR EACH TERM
- $\theta$ : ANGLE FORMED BY THREE CONSECUTIVE COARSE-GRAINED PARTICLES, $\tau$ : DIHEDRAL ANGLE FORMED BY FOUR CONSECUTIVE COARSE-GRAINED PARTICLES
- $\theta_0$: DESIRED VALUE OF ANGLE, $\tau_0$: DESIRED VALUE OF DIHEDRAL ANGLE
- $r_{ij}$: DISTANCE BETWEEN i-TH COARSE-GRAINED PARTICLE AND j-TH COARSE-GRAINED PARTICLE, $\sigma_{ij}$: DISTANCE IN NATURAL STATE BETWEEN i-TH COARSE-GRAINED PARTICLE AND j-TH COARSE-GRAINED PARTICLE
- $r_{1N}$: DISTANCE BETWEEN COARSE-GRAINED PARTICLES AT TWO ENDS, $R_{th}$: DESIRED VALUE FOR DISTANCE BETWEEN COARSE-GRAINED PARTICLES AT TWO ENDS

# FIG. 6

510

**ENERGY COMPUTATION**

$$E = \sum_{angles} K_\theta \cdot (\theta - \theta_0) + \sum_{dihedral} K_\tau \cdot \left[1 + cos\big(N(\tau - \tau_0)\big)\right] + \sum_{i<j-3} \left[5\left(\frac{\sigma_{ij}}{r_{ij}}\right)^{12} - 6\left(\frac{\sigma_{ij}}{r_{ij}}\right)^{10}\right] + K_{end} \cdot (R_{th} - r_{1N})^4$$

ANGLE · DIHEDRAL ANGLE · REPULSIVE FORCE OR ATTRACTIVE FORCE · DISTANCE BETWEEN TWO ENDS WHEN HAVING CYCLIC STRUCTURE

610

**COMPUTATION OBJECT**

COARSE-GRAINED PARTICLE i+1

COARSE-GRAINED PARTICLE i+1

$\theta$

$\theta'$

COARSE-GRAINED PARTICLE i

COARSE-GRAINED PARTICLE i

COARSE-GRAINED PARTICLE i-1

COARSE-GRAINED PARTICLE i-1

COMPUTE DIFFERENCE AT PORTION THAT CHANGES DUE TO STATE TRANSITION

620

**COMPUTATION OBJECT**

COARSE-GRAINED PARTICLE i

COARSE-GRAINED PARTICLE i+1

COARSE-GRAINED PARTICLE i+1

$\tau'$

COARSE-GRAINED PARTICLE i

$\tau$

PLANE A

PLANE B

PLANE A

PLANE B'

COARSE-GRAINED PARTICLE i-1

COARSE-GRAINED PARTICLE i-1

COARSE-GRAINED PARTICLE i-2

COARSE-GRAINED PARTICLE i-2

COMPUTE DIFFERENCE AT PORTION THAT CHANGES DUE TO STATE TRANSITION

# FIG. 7

ENERGY COMPUTATION — 510

$$E = \sum_{angles} K_\theta \cdot (\theta - \theta_0) + \sum_{dihedral} K_\tau \cdot \left[1 + cos(N(\tau - \tau_0))\right] + \sum_{i<j-3} \left[5\left(\frac{\sigma_{ij}}{r_{ij}}\right)^{12} - 6\left(\frac{\sigma_{ij}}{r_{ij}}\right)^{10}\right] + K_{end} \cdot (R_{th} - r_{1N})^4$$

ANGLE    DIHEDRAL ANGLE    REPULSIVE FORCE OR ATTRACTIVE FORCE    DISTANCE BETWEEN TWO ENDS WHEN HAVING CYCLIC STRUCTURE

COMPUTATION OBJECT — 710

COARSE-GRAINED PARTICLE i+1

COARSE-GRAINED PARTICLE i+1    GROUP B

GROUP A    GROUP B

GROUP A

COARSE-GRAINED PARTICLE i

COARSE-GRAINED PARTICLE i-1

COARSE-GRAINED PARTICLE i

COARSE-GRAINED PARTICLE i-1

COMPUTE FOR COMBINATION WITH COARSE-GRAINED PARTICLE BELONGING TO DIFFERENT GROUP

COMPUTATION OBJECT — 720

WHEN HAVING CYCLIC STRUCTURE

EP 4 293 674 A1

# FIG. 8

800

420

RELATIVE COORDINATES

<0>: (-1, -1, 0)、<1>: (-1, 1, 0)、<2>: (1, -1, 0)、<3>: (1, 1, 0)
<4>: (-1, 0, -1)、<5>: (-1, 0, 1)、<6>: (1, 0, -1)、<7>: (1, 0, 1)
<8>: (0, -1, -1)、<9>: (0, -1, 1)、<10>: (0, 1, -1)、<11>: (0, 1, 1)

810 TERMINAL DEVICE

811 STATE VARIABLE INPUT UNIT

812 ENERGY MODEL INPUT UNIT

813 SEARCH INSTRUCTION UNIT

814 EXECUTION INSTRUCTION UNIT

INITIAL STATE AND RELATIVE COORDINATES

NUMBER OF SEARCHES OR VARIABLES TO BE SEARCHED FOR

210

820 ISING DEVICE

SEARCH INSTRUCTION

SEARCH RESULT (MINIMUM ENERGY AND STATE VARIABLES)

510

$$E = \sum_{angles} K_\theta \cdot (\theta - \theta_0) + \sum_{dihedral} K_\tau \cdot [1 + cos(N(\tau - \tau_0))]$$
$$+ \sum_{i<j-3} \left[ 5\left(\frac{\sigma_{ij}}{r_{ij}}\right)^{12} - 6\left(\frac{\sigma_{ij}}{r_{ij}}\right)^{10} \right] + K_{end} \cdot (R_{th} - r_{1N})^4$$

E●

FIG. 9

800

810 TERMINAL DEVICE

SEARCH INSTRUCTION

SEARCH RESULT (MINIMUM ENERGY VALUE AND STATE VARIABLES)

[0, 1, 9, 6]

STATE VARIABLES (CURRENT)

910 SEARCH UNIT

ENERGY VALUE

STATE VARIABLES (AFTER STATE TRANSITION)

ISING DEVICE 820

950 ENERGY VALUE STORAGE UNIT

960 STATE VARIABLE STORAGE UNIT

920 OPTIMAL SOLUTION SPECIFYING UNIT

[0, 1, 4, 6]

970 RELATIVE COORDINATE STORAGE UNIT

940 ENERGY COMPUTATION UNIT

930 STATE CONVERSION UNIT

510

$$E = \sum_{angles} K_\theta \cdot (\theta - \theta_0) + \sum_{dihedral} K_\tau \cdot \left[1 + \cos\left(N(\tau - \tau_0)\right)\right]$$
$$+ \sum_{i<j-3} \left[5\left(\frac{\sigma_{ij}}{r_{ij}}\right)^{12} - 6\left(\frac{\sigma_{ij}}{r_{ij}}\right)^{10}\right] + K_{end} \cdot (R_{th} - r_{1N})^4$$

COARSE-GRAINED PARTICLE 0:
COARSE-GRAINED PARTICLE 1: (0, 0, 0) + (-1, -1, 0) =
COARSE-GRAINED PARTICLE 2: (-1, -1, 0) + (-1, 1, 0) =
COARSE-GRAINED PARTICLE 3: (-2, 0, 0) + (-1, 0, -1) =
COARSE-GRAINED PARTICLE 4: (-3, 0, -1) + (1, 0, -1) =

(0, 0, 0)
(-1, -1, 0)
(-2, 0, 0)
(-3, 0, -1)
(-2, 0, -2)

420 RELATIVE COORDINATES

<0>: (-1, -1, 0)、<1>: (-1, 1, 0)、<2>: (1, -1, 0)、<3>: (1, 1, 0)
<4>: (-1, 0, -1)、<5>: (-1, 0, 1)、<6>: (1, 0, -1)、<7>: (1, 0, 1)
<8>: (0, -1, -1)、<9>: (0, -1, 1)、<10>: (0, 1, -1)、<11>: (0, 1, 1)

EP 4 293 674 A1

# FIG. 10

TERMINAL DEVICE ⟋810

PROCESSOR ⟋1001

MEMORY ⟋1002

AUXILIARY STORAGE DEVICE ⟋1003

⟋1007

I/F DEVICE ⟋1004

COMMUNICATION DEVICE ⟋1005

DRIVE DEVICE ⟋1006

OPERATION DEVICE ⟋1010

OUTPUT DEVICE ⟋1020

RECORDING MEDIUM ⟋1030

EP 4 293 674 A1

# FIG. 11

```
         ┌────────────────────────────────┐
         │   START OF STABLE STRUCTURE     │
         │        SEARCH PROCESS           │
         └────────────────────────────────┘
                        │
                        ▼
   ┌──────────────────────────────────────┐
   │   SET NUMBER OF RESIDUES AND RESIDUE  │────  S1101
   │             SEQUENCE                  │
   └──────────────────────────────────────┘
                        │
                        ▼
   ┌──────────────────────────────────────┐
   │  RANDOMLY DESIGNATE INITIAL STATE OF N│
   │   COARSE-GRAINED PARTICLES (RANDOMLY  │────  S1102
   │    DESIGNATE N-1 STATE VARIABLES)     │
   └──────────────────────────────────────┘
                        │
                        ▼
   ┌──────────────────────────────────────┐
   │   INPUT RELATIVE COORDINATES, ENERGY  │
   │  COMPUTATION FORMULA, AND NUMBER OF   │────  S1103
   │              SEARCHES                 │
   └──────────────────────────────────────┘
                        │
                        ▼
   ┌──────────────────────────────────────┐
   │ │         SEARCH PROCESS          │ │ │────  S1104
   └──────────────────────────────────────┘
                        │
                        ▼
   ┌──────────────────────────────────────┐
   │  OUTPUT MINIMUM ENERGY VALUE AND STATE│────  S1105
   │             VARIABLES                 │
   └──────────────────────────────────────┘
                        │
                        ▼
         ┌────────────────────────────────┐
         │    END OF STABLE STRUCTURE      │
         │        SEARCH PROCESS           │
         └────────────────────────────────┘
```

# FIG. 12

```
┌─────────────────────────┐
│   START OF SEARCH       │
│   PROCESS               │
└─────────────────────────┘
            │
            ▼
┌──────────────────────────────────────┐
│ COMPUTE ENERGY VALUE E FOR INITIAL    │  ～ S1201
│ STATE AND STORE minE = E              │
└──────────────────────────────────────┘
            │                              ②
            ▼
┌──────────────────────────────────────┐
│ RANDOMLY SELECT ANY ONE OF N-1 STATE  │  ～ S1202
│ VARIABLES AND SET index = X           │
└──────────────────────────────────────┘
            │
            ▼
┌──────────────────────────────────────┐
│ UPDATE STATE VARIABLE X               │  ～ S1203
└──────────────────────────────────────┘
            │
            ▼
┌──────────────────────────────────────┐
│ UPDATE COORDINATES OF i+1-TH COARSE-  │  ～ S1204
│ GRAINED PARTICLE                      │
└──────────────────────────────────────┘
            │
            ▼
┌──────────────────────────────────────┐
│ UPDATE COORDINATES OF i+2-TH TO N-TH  │  ～ S1205
│ COARSE-GRAINED PARTICLES (MOVE i+2-TH │
│ TO N-TH COARSE-GRAINED PARTICLES IN   │
│ PARALLEL IN ACCORDANCE WITH MOVEMENT  │
│ OF i+1-TH COARSE-GRAINED PARTICLE)    │
└──────────────────────────────────────┘
            │
            ▼
┌──────────────────────────────────────┐
│ CALCULATE ENERGY VALUE E' BASED ON    │  ～ S1206
│ CURRENT COORDINATES                   │
└──────────────────────────────────────┘
            │
            ▼
           ①
```

# FIG. 13

① → S1301

IS (E′ - E) < -Tlog(a) TRUE? — NO → S1303 REJECT STATE VARIABLES AFTER TRANSITION

YES → S1302 ADOPT STATE VARIABLES AFTER TRANSITION AND UPDATE ENERGY VALUE

→ S1304 IS E < minE TRUE? — NO

YES → S1305 STORE E = minE AND STORE CORRESPONDING STATE VARIABLES AS OPTIMAL STATE VARIABLES

→ S1306 HAS NUMBER OF SEARCHES BEEN REACHED? — NO → ②

YES → RETURN

# FIG. 14

1410

| ITEM | FACE-CENTERED CUBIC | NIGHT WALK |
|---|---|---|
| NUMBER OF Bits | 133 Kbit | 616 Kbit |
| TOTAL NUMBER OF STATES | $2.4 \times 10^{63}$ PATTERNS | $2.1 \times 10^{75}$ PATTERNS |
| RAM CAPACITY | 66.3 Gbyte | 1412.6 Gbyte |

1420

| ITEM | FACE-CENTERED CUBIC | NIGHT WALK |
|---|---|---|
| NUMBER OF Bits | 228 Kbit | 456 Kbit |
| TOTAL NUMBER OF STATES | $3.2 \times 10^{20}$ PATTERNS | $1.7 \times 10^{26}$ PATTERNS |
| RAM CAPACITY | 1.4 Kbyte | 1.4 Kbyte |

Europäisches Patentamt
European Patent Office
Office européen des brevets

# EUROPEAN SEARCH REPORT

Application Number

EP 23 15 5997

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TOMAS BABEJ ET AL: "Coarse-grained lattice protein folding on a quantum annealer", ARXIV.ORG, CORNELL UNIVERSITY LIBRARY, 201 OLIN LIBRARY CORNELL UNIVERSITY ITHACA, NY 14853, 2 November 2018 (2018-11-02), XP080941413, * the whole document * | 1-12 | INV. G16C20/30 G16B15/00 G16C20/40 |
| X | Ryan Babbush ET AL: "Construction of Energy Functions for Lattice Heteropolymer Models: Efficient Encodings for Constraint Satisfaction Programming and Quantum Annealing" In: "Advances in Chemical Physics: Volume 155", 11 April 2014 (2014-04-11), John Wiley & Sons, Inc., Hoboken, New Jersey, XP055692281, ISBN: 978-1-118-75577-8 pages 201-244, DOI: 10.1002/9781118755815.ch05, * the whole document * | 1-12 | |
| X | WONG RENATA ET AL: "Quantum Speedup for Protein Structure Prediction", IEEE TRANSACTIONS ON NANOBIOSCIENCE, IEEE SERVICE CENTER, PISCATAWAY, NY, US, vol. 20, no. 3, 10 March 2021 (2021-03-10), pages 323-330, XP011863721, ISSN: 1536-1241, DOI: 10.1109/TNB.2021.3065051 [retrieved on 2021-06-30] * the whole document * | 1-12 | TECHNICAL FIELDS SEARCHED (IPC) G16C G16B |

-/--

The present search report has been drawn up for all claims

| Place of search | Date of completion of the search | Examiner |
|---|---|---|
| The Hague | 20 October 2023 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

**EP 23 15 5997**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BENÍTEZ CÉSAR MANUEL VARGAS ET AL: "Protein structure prediction with the 3D-HP side-chain model using a master-slave parallel genetic algorithm", BRAZILIAN COMPUTER SOCIETY. JOURNAL, vol. 16, no. 1, 24 April 2010 (2010-04-24), pages 69-78, XP093093325, BR ISSN: 0104-6500, DOI: 10.1007/s13173-010-0002-6 * the whole document * | 1-12 | |
| X | SEAN MULLANE: "Protein folding in the modern era: a pedestrian's guide", ARXIV.ORG, CORNELL UNIVERSITY LIBRARY, 201 OLIN LIBRARY CORNELL UNIVERSITY ITHACA, NY 14853, 10 July 2020 (2020-07-10), XP081712103, * page 14 – page 17 * | 1-12 | |

TECHNICAL FIELDS
SEARCHED      (IPC)

The present search report has been drawn up for all claims

| Place of search | Date of completion of the search | Examiner |
|---|---|---|
| The Hague | 20 October 2023 | Denoual, Matthieu |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

 

& : member of the same patent family, corresponding
     document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019230303 A
- JP 2020194487 A
- JP 2019179304 A
- JP 2021082165 A